# EUROPÄISCHE PATENTSCHRIFT

(11) **EP 1 550 467 B1**
(45) Veröffentlichungstag und Bekanntmachung des Hinweises auf die Patenterteilung: **27.02.2008**
(21) Anmeldenummer: 03028828.6
(22) Anmeldetag: 15.12.2003
(51) Int. Cl.: A61L 2/28, G01N 31/22, C12Q 1/22

(54) **Prüfkörper zur Überprüfung der Penetrationseigenschaften eines Sterilisationsmittels in Sterilisationsprozessen**
Test device for checking the permeation properties of a sterilising agent in sterilisation processes
Dispositif de test pour contrôler les propriétés de pénétration d'un agent de stérilisation dans des procédés de stérilisation

(43) Veröffentlichungstag der Anmeldung: 06.07.2005
(73) Patentinhaber: Kaiser, Ulrich, Dr., D-61479 Glashütten (DE)
(72) Erfinder: Kaiser, Ulrich, Dr., D-61479 Glashütten (DE)
(74) Vertreter: Walkenhorst, Andreas

(56) Entgegenhaltungen:
- EP-A- 0 628 814
- DE-A- 19 724 158
- DE-U- 9 104 824
- US-A- 4 914 034

## Beschreibung

Die Erfindung bezieht sich auf einen Prüfkörper in Hohlkörperbauweise nach Anspruch 1. Sie betrifft weiterhin ein Verfahren zur Herstellung eines derartigen Prüfkörpers. Derartige Prüfkörper sind beispielsweise aus der EP 0 628 814 A1 oder aus der EP 1 172 117 A2 bekannt.

Bei aseptischen Anwendungen wie bei Operationen im Krankenhaus ist die Verwendung von sterilen Werkzeugen, Instrumenten oder Materialien zwingend erforderlich. Bei einer dazu notwendigen Sterilisation wird üblicherweise das sterilisierende Medium, wie beispielsweise Dampf, Formaldehyd, Ethylenoxid, Wasserstoffperoxid und/oder Ozon, über die Gasphase auf die Oberfläche des zu sterilisierenden Instruments übertragen, um die totale Abtötung vorhandener Keime zu gewährleisten. Zu diesem Zweck kommen normalerweise Sterilisatoren mit Sterilisationskammern zum Einsatz, in die die zu sterilisierenden Instrumente oder Materialien - im Regelfall verpackt - eingelegt werden. Zur eigentlichen Sterilisation wird die Sterilisationskammer mit gasförmigem Sterilisiermittel - auch als sterilisierendes Agens bezeichnet - geflutet, wobei zuvor die Entfernung der Luftatmosphäre gewährleistet sein muss. Das Sterilisiermittel soll die Oberflächen der zu sterilisierenden Instrumente oder Materialien kontaktieren, so dass die erwünschte Keimabtötung eintritt.

Da die vollständige Sterilisation der Güter an allen Stellen nur dann sichergestellt ist, wenn das Sterilisiermittel auch alle inneren Oberflächen erreicht, z. B. poröse Güter oder Hohlkörper ist zu Beginn des Sterilisationsprozesses die Entfernung der im Innern der Güter und im Innern des Sterilisierraums befindlichen Luft durch ein geeignetes Entlüftungsverfahren zu gewährleisten. Anschließend wird die Sterilisationskammer mit dem Sterilisiermittel geflutet, um im Innern der Sterilisationskammer alle Oberflächen der Gerätschaften mit dem Sterilisiermittel zu erreichen. Dies ist nur möglich, wenn die vollständige Penetration des Sterilisiermittels an alle Oberflächen gewährleistet ist.

Als problematisch bei derartigen Sterilisationsmaßnahmen hat sich jedoch die Komplexität beim strukturellen Aufbau medizinischer Instrumente erwiesen. Gerade bei medizinischen Instrumenten kommen nämlich zunehmend Gerätschaften zum Einsatz, bei denen Röhren oder Schläuche mit relativ großer Länge und vergleichsweise geringem freien Querschnitt verwendet werden, so dass ein zuverlässiges Benetzen des Sterilisiermittels von sämtlichen inneren Oberflächen erschwert ist, wenn sich dort andere Gase befinden. Des Weiteren können Materialien oder Güter mit komplexer Innenoberfläche, wie beispielsweise Textilpakete, zu sterilisieren sein. In derartigen Fällen können eventuell vorhandene Ansammlungen von Restluft oder anderen nicht kondensierbaren Gasen (NKG) den durchgängigen Kontakt der behandlungsbedürftigen Oberfläche ganz oder teilweise verhindern. Die vollständige Sterilisation ist hingegen nur dann sichergestellt, wenn die sich im Innern der Güter befindliche Luft zu Beginn der Sterilisationsmaßnahme vollständig entfernt wird, keine Luft durch Undichtigkeiten während der Vakuumphase eindringt und/oder keine nicht kondensierbare Gase in die Sterilisationskammer mit dem Sterilisiermittel eingetragen werden, damit das Sterilisiermittel an alle behandlungsbedürftigen Oberflächen gelangt.

Da zudem die Sterilität von Instrumenten vor ihrem Gebrauch nicht direkt geprüft werden kann, sind bei der Inbetriebnahme von Sterilisationsprozessen die Validierung und während des Betriebs des Sterilisationsprozesses Routinekontrollen notwendig. Dazu kommen Detektionssysteme zum Einsatz, die den Sterilisationserfolg nachweisen müssen. Beispielsweise können Chemo-Indikatoren zum Einsatz kommen, die im Falle einer Benetzung ihrer Oberfläche durch das Sterilisiermittel unter Temperatureinwirkung, wie beispielsweise Dampf, ihre Farbe ändern, so dass direkt erkennbar ist, dass an der Stelle des Chemo-Indikators ein Kontakt tatsächlich stattgefunden hat. Alternativ oder zusätzlich können auch Bio-Indikatoren eingesetzt werden, die in Form von Streifen, Suspensionen oder selbst entwickelnden Keimkulturen oder Gemischen verschiedener Keimkulturen zum Einsatz kommen, bei denen nach der Durchführung der Sterilisationsmaßnahme überprüft wird, ob alle Keime vollständig inaktiviert wurden.

Bei der Verwendung derartiger Indikatoren wird überprüft, ob am tatsächlichen Einsatzort des Indikators innerhalb der Sterilisationskammer eine aktive Benetzung der Indikatoroberfläche mit Sterilisiermittel eingetreten ist. Bei der Verwendung dieser Indikatoren ist jedoch ein direkter Nachweis des Sterilisationserfolgs auch an vergleichsweise unzugänglichen Innenoberflächen komplexer Instrumente nicht möglich, da diese an den kritischen Stellen nicht platziert werden können. Daher werden gemeinsam mit den behandlungsbedürftigen Gütern Prüfkörpersysteme mit sterilisiert, durch die der Sterilisationserfolg ermittelt wird. Beispielsweise wurde für die Sterilisationsmaßnahme bei Textilien oder anderen Materialien von Bowie und Dick (Bowie, I. W., e. a., The Bowie + Dick autoclave tape test, Lancet I, 1963, S. 585-587) ein Standardtest angegeben, bei dem in einem Wäschepaket von 6,6 kg Gewicht zentral ein Chemo-Indikatorblatt von DIN-A-4-Größe platziert wird. Allerdings ist dieser Standardtest aufgrund der Wäschequalität, Wäschehistorie und Packungsindividualität nicht immer reproduzierbar und hat andere Penetrationseigenschaften als Hohlkörper.

Alternativ können so genannte Prüfkörper oder Prüfkörpersysteme eingesetzt werden. Bei einem derartigen Prüfkörpersystem, wie es beispielsweise in der EP 0 628 814 A1 oder in der EN 867-5 beschrieben ist, wird die unzugängliche Innenoberfläche komplexer Instrumente durch ein geeignetes Modell nachgebildet, über das der Erfolg der Penetrationsvorgänge in analoger Weise auch für komplexe Instrumente überprüft werden kann.

Bei diesen bekannten, auch als "Process Challenge Device" (PCD) bezeichneten Prüfkörpersystemen in Hohlkörperbauweise ist ein zum Nachweis eines erfolgten Kontakts durch das Sterilisiermittel geeignet ausgebildeter Detektor gaseingangsseitig mit einem in seiner Länge geeignet gewählten, an seinem Einlassende offen gehaltenen Schlauchmodell verbunden. Das Hohlkörpersystem simuliert dabei das Verhalten ähnlich gestalteter Instrumente, die zur Sterilisation vorgesehen sind, wobei insbesondere bei alternierendem Medienaustausch nach Art eines fraktionierten Vakuums und/oder bei der Kondensation von Dampf eventuell noch vorhandene Restluft oder andere nicht kondensierbare Gase am Schlauchende und somit im Bereich des Detektors aufkonzentriert werden. Der Schlauch wirkt somit als Gassammelraum für Restluft oder andere nicht kondensierbare Gase, wobei der Detektor über diesen Gassammelraum durch Einlegen in die Sterilisationskammer mit dem Sterilisationsraum verbunden ist.

Falls bei der Verwendung eines derartigen Systems ein Kontakt des am Schlauchende angeordneten Detektors mit Sterilisiermittel festgestellt wird, kann davon ausgegangen werden, dass - gegebenenfalls unter Berücksichtigung eines geeignet gewählten Sicherheitsaufschlags in Bezug auf die Penetrationseigenschaften - in den zu sterilisierenden Instrumenten auch an deren unzugänglichsten Stellen ihrer Innenoberfläche ein Kontakt mit Sterilisiermittel erfolgt sein muss. Ein derartiges Schlauchmodell als Prüfkörper, das als Detektor beispielsweise Bio- oder Chemo-Indikatoren aufnehmen kann, ist auch in der Euronorm EN 867-5 bei der Überprüfung von Sterilisationsprozessen beschrieben. Bei noch komplexeren zu sterilisierenden Gütern können in ihrer Dimensionierung geeignet angepasste Prüfkörper anderer Bauart eingesetzt werden, wie dies beispielsweise in den Euronormen EN 285, EN 14180, EN 1422 oder EN 867-5 beschrieben ist.

Der Einsatz derartiger Prüfkörper ermöglicht auch, unter bestimmten Randbedingungen physikalische Messmethoden einzusetzen. Beispielsweise ist aus der EP 1 172 117 A2 ein Prüfsystem für Sterilisationsmaßnahmen mit einem Prüfkörper bekannt, bei dem als Nachweis für eine erfolgte Benetzung einer Innenoberfläche durch Sterilisiermittel die mit der lokalen Kondensation von Dampf an der zu überwachenden Stelle verbundene lokale Temperaturänderung gemessen wird. Der in diesem System eingesetzte Prüfkörper ist demzufolge hinsichtlich seiner Wärmeleiteigenschaften besonders geeignet dimensioniert.

Die Verwendung der genannten Hohlkörpersysteme auf der Basis von Prüfkörpern bei der Überprüfung der Penetrationseigenschaften eines Sterilisiermittels in Sterilisationsprozessen ist grundsätzlich vergleichsweise günstig, da durch geeignete Dimensionierung des Gassammelraums und gegebenenfalls des Detektorraums eine Vielzahl von Prüfbedingungen simulierbar oder einstellbar sind. Eine besonders einfach gehaltene, vielseitig einsetzbare Ausgestaltung derartiger Prüfkörper in Hohlkörperbauweise ist daher wünschenswert.

Der Erfindung liegt daher die Aufgabe zugrunde, einen Prüfkörper der oben genannten Art anzugeben, mit dem besonders zuverlässige Prüfungsergebnisse bei einem besonders gering gehaltenen Aufwand erreichbar sind. Des Weiteren soll ein Verfahren zur Herstellung eines derartigen Prüfkörpers angegeben werden.

Diese Aufgabe wird erfindungsgemäß gelöst durch die Merkmale des Anspruchs 1.

Die Auslegung als Einwegprodukt für den Prüfkörper spezifiziert dabei insbesondere, dass dieser hinsichtlich der Wahl der eingesetzten Materialien und/oder der eingesetzten Herstellungs- oder Bearbeitungsverfahren besonders kostengünstig ausgeführt und lediglich zum einmaligen Gebrauch vorgesehen ist. Im Gegensatz zu Prüfkörpern in Hohlkörperbauweise, die für Mehrfacheinsätze vorgesehen sind und somit notwendigerweise ein wiederverwendbares Aufnahmesystem für den Indikator umfassen, ist der Erfindungsgemäße, als Einwegprodukt ausgelegte Prüfkörper für seine irreversible Zerstörung bei der Entnahme des Indikators ausgelegt.

Die Erfindung geht von der Überlegung aus, dass die Zuverlässigkeit der Prüfungsergebnisse bei der Verwendung von Prüfkörpern in Hohlkörperbauweise besonders hoch gehalten werden kann, indem denkbare Verschmutzungen, Verunreinigungen und insbesondere auch Reste von Kondensat oder Sterilisiermittel aus einer vorangegangenen Verwendung im Prüfkörpersystem konsequent vermieden werden. Gerade der mehrfache Einsatz eines derartigen Prüfkörpers könnte aber zur Verunreinigung oder Verschmutzung des Gesamtsystems beitragen, so dass vor einer erneuten Verwendung des Prüfkörpersystems eine vergleichsweise aufwendige Endreinigung und Prüfung auf Sauberkeit und Dichtheit notwendig ist. Demgegenüber gewährleistet ein lediglich einmaliger Einsatz eines derartigen Prüfkörpersystems besonders hohen Schutz vor den oben genannten Veränderungen. Um zudem noch die flexible Anpassung an individuell vorgebbare Prüfbedingungen einerseits sowie eine besonders kostengünstige Ausführung des Prüfkörpers andererseits zu ermöglichen, ist der Prüfkörper im Hinblick auf die konstruktive Auslegung seiner Komponenten und auch die Wahl der eingesetzten Materialien konsequent als Einwegprodukt ausgelegt.

Eine besonders einfache und kostengünstige Herstellung ist dabei erreicht, indem der verwendete Indikator an einem Schlauchende eingebettet und das Schlauchende zur Bildung des Detektorraums anschließend verschweißt wird.

Der Schlauch kann dabei zweckmäßigerweise für eine optische Auswertung der Prüfergebnisse in vorteilhafter Ausgestaltung aus transparentem Kunststoff gebildet sein.

Um eine besonders flexible und zuverlässige Vorgabe selektiver Prüfbedingungen zu ermöglichen, ist der Gassammelraum vorteilhafterweise mit einer Wärme isolierenden Ummantelung versehen.

Eine besonders kompakte Bauweise ist erreichbar, indem der Gassammelraum vorzugsweise in der Art einer Spirale ausgebildet ist, indem vorzugsweise ein Plastikteil tiefgezogen oder gespritzt wird. Insbesondere bei der Verwendung eines Schlauchs zur Bildung des Detektorraums und insbesondere des Gassammelraums kann der Schlauch dabei zu einer Helix gerollt und mit Kabelbindern oder sonstigen Verbindungsteilen zusammengehalten sein.

Um eine besonders einfache Entnahme des Indikators nach durchgeführter Prüfung zu ermöglichen, ist der Detektorraum vorteilhafterweise mit einer Sollbruchstelle versehen. Gerade durch die Zerstörung des Detektorraums bei der Entnahme des Indikators ist dabei gewährleistet, dass im Sinne des bestimmungsgemäßen Gebrauchs des Prüfkörpers eine lediglich einmalige Verwendung eingehalten wird.

Eine besonders hohe Nachweisempfindlichkeit des Prüfkörpers ist erreichbar, indem der Gassammelraum in besonders vorteilhafter Ausgestaltung mehrstufig ausgeführt ist, wobei der Querschnitt und/oder das Volumen jeder Stufe zwischen benachbarten Stufen in Richtung zum Detektorraum hin abnehmen. Wie sich nämlich überraschenderweise herausgestellt hat, ermöglicht eine derartige Auslegung die selektive Aufkonzentration noch vorhandener Restluft oder Restgase im Gassammelraum in unmittelbarer Nachbarschaft zum Detektorraum, so dass eine besonders hohe Verstärkung der Nachweisempfindlichkeit erreichbar ist.

Als Indikator kann ein so genannter physikalischer Indikator, also ein System zur Ermittlung physikalischer Kennwerte wie beispielsweise ein Feuchtigkeits-, Temperatur-, Druck- und/oder Ultraschallsensor, der sich im Detektorraum befindet, zum Einsatz kommen, der bedarfsweise auch als so genannter Daten-Logger für eine drahtlose Übermittlung der aufgenommenen Daten ausgestaltet sein kann. Auch können beispielsweise Festkörper, wie z. B. Salze, eingesetzt sein, die sich bei Anwesenheit des Sterilisiermittels physikalisch verändern, also beispielsweise ihren Schmelzpunkt oder ihre Farbe ändern. Des Weiteren kann als Indikator ein so genannter Chemo-Indikator, der bei Kontakt mit dem eingesetzten Sterilisiermittel seine Farbe ändert, oder ein Bio-Indikator, beispielsweise in Form von Indikatorstreifen und/oder selbstentwickelnden Bio-Indikatoren, vorgesehen sein.

Eine konsequente Ausnutzung der Auslegung des Prüfkörpers als Einwegprodukt hinsichtlich gering gehaltenem Herstellungsaufwand ist aber erreichbar, indem als Indikator in besonders vorteilhafter Ausgestaltung auf eine transparente Innenwand des den Detektorraum bildenden Gehäuses aufgebrachte Indikatorfarbe vorgesehen ist. Bei dieser Ausführung ist der Indikator mechanisch fest mit Teilen des den Detektorraum bildenden Gehäuses integriert, so dass gesonderte Stützoder Tragstrukturen für den Indikator als solchen entfallen können. Aufgrund der Auslegung des Prüfkörpers als Einwegprodukt kann nach dessen Einsatz der Indikator gemeinsam mit den ihn tragenden Gehäuseteilen des Detektorraums entnommen und geeignet begutachtet und eventuell archiviert werden.

Bezüglich des Verfahrens zur Herstellung eines Prüfkörpers der beschriebenen Art wird die genannte Aufgabe gelöst, indem auf die Innenwand eines zur Bereitstellung eines Detektorraums vorgesehenen Schlauchstücks eine Suspension eines Chemo- oder Bio-Indikators aufgebracht und anschließend getrocknet wird. Bereits herstellungsbedingt ist der dabei erhältliche Prüfkörper als Einwegprodukt ausgelegt, wobei auf diese Weise eine besonders einfach gehaltene und somit kostengünstige Herstellung des Prüfkörpers gewährleistet ist.

Die mit der Erfindung erzielten Vorteile bestehen insbesondere darin, dass durch die konsequente Auslegung gerade eines in Hohlkörperbauweise ausgeführten Prüfkörpers als Einwegprodukt die Beeinträchtigung der Prüfungsergebnisse durch Rückstände des Sterilisiermittels aus vorangegangenen Prozessen, Verunreinigungen oder Verschmutzungen in besonderem Maße gering gehalten werden können. Somit ist eine besonders hohe Zuverlässigkeit und Reproduzierbarkeit bei der Ermittlung der Überprüfungsergebnisse erreichbar. Durch die Ausführung des Prüfkörpersystems in Hohlkörperbauweise als Einwegprodukt können je nach Bedarf und Konstruktion besonders hohe Anforderungen an die Penetrationseigenschaften eines Sterilisationsprozesses gestellt werden, so dass reproduzierbar sichergestellt werden kann, dass die zunehmend eingesetzten Hohlkörperinstrumente, beispielsweise für minimal-invasiv-chirurgische Anwendungen und/oder lange Schläuche zum Absaugen und Betreiben von pneumatischen Bohrmaschinen, mit besonders hoher Zuverlässigkeit sterilisiert werden. Gerade durch die Ausgestaltung eines in Hohlkörperbauweise ausgeführten Prüfkörpers als Einwegprodukt kann für derartig komplexe hohle Instrumente und Schläuche eine besonders hohe Sterilisationssicherheit gewährleistet werden.

Ein Ausführungsbeispiel der Erfindung wird anhand einer Zeichnung näher erläutert. Darin zeigen:
- Fig. 1, 2: jeweils schematisch einen Prüfkörper in Hohlkörperbauweise,
- Fig. 3: ein Aufbauschema für einen Prüfkörper, und
- Fig. 4: einen Prüfkörper zur Überprüfung der Penetrationseigenschaften eines Sterilisiermittels.

Gleiche Teile sind in allen Figuren mit den selben Bezugszeichen versehen.

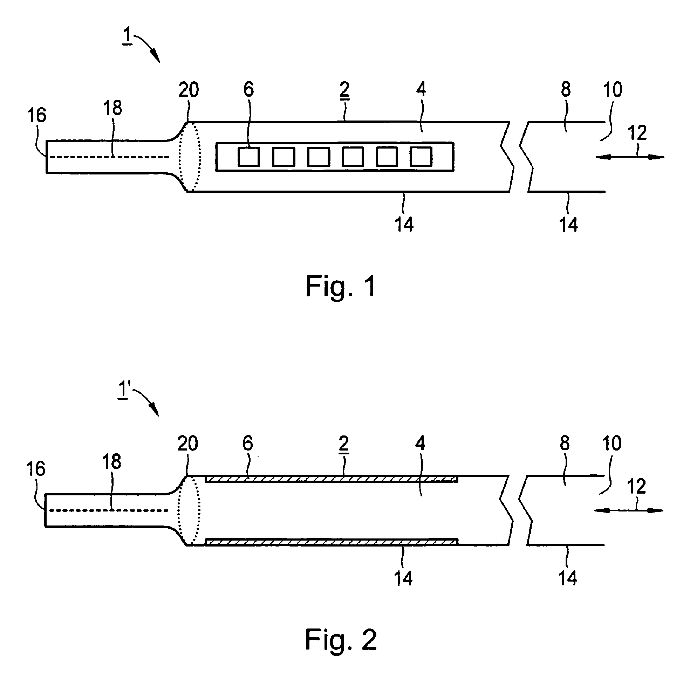

Der Prüfkörper 1 gemäß Fig. 1 ist zur Überprüfung der Penetrationseigenschaften eines Sterilisiermittels ausgelegt. Die beim Einsatz des Prüfkörpers 1 gewonnenen Erkenntnisse können insbesondere zur Verifizierung oder Überprüfung von Sterilisationsmaßnahmen in einem Dampfsterilisationsverfahren herangezogen werden. Bei einer derartigen Dampfsterilisation werden die zu sterilisierenden Instrumente oder Materialien in eine nicht näher dargestellte Sterilisationskammer eingebracht. Zur Sterilisation wird die Sterilisationskammer zunächst entlüftet. Der Entlüftungsprozess kann dadurch Strömungsverfahren, Über- oder Unterdruck-Entlüftungszyklen oder deren Kombination erfolgen.

Um bei einem derartigen, auf fraktioniertem Vakuum beruhenden Entlüftungsverfahren nachweisen zu können, dass bei den zu sterilisierenden Instrumenten und Materialien auch tatsächlich sämtliche inneren Oberflächen mit Sterilisiermittel benetzt worden sind und somit die erforderliche vollständige Penetration stattgefunden hat, wird der Prüfkörper 1 beispielsweise zur Validierung der Sterilisationsmaßnahme bei ihrer Inbetriebnahme oder bei Routinekontrollen während des Sterilisationsprozesses mit in der Sterilisationskammer eingesetzt. Der Prüfkörper 1 umfasst einen Detektor 2, der für den gezielten Nachweis ausgelegt ist, dass eine Benetzung durch Sterilisiermittel tatsächlich stattgefunden hat.

Der Prüfkörper 1 ist dafür ausgebildet, die ordnungsgemäße Benetzung auch vergleichsweise schwer zugänglicher innerer Oberflächen von Instrumenten oder Materialien geeignet nachzubilden. Dazu ist der Prüfkörper 1 in so genannter Hohlkörperbauweise ausgeführt. In dieser Hohlkörperbauweise umfasst der Detektor 2 einen in einem Detektorraum 4 angeordneten Indikator 6, der zum Nachweis einer Benetzung durch das Sterilisiermittel geeignet ausgeführt ist. Dem Detektorraum 4 ist gasseitig ein Gassammelraum 8 vorgeschaltet, dessen Endmündung 10 offen gehalten ist. Somit ist der Detektor 2, wie durch den Doppelpfeil 12 angedeutet, über den Gassammelraum 8 gasseitig mit der Sterilisationskammer verbunden.

Der Prüfkörper 1 ist insbesondere für eine Anwendung bei einem dampfbasierten Sterilisationsverfahren unter Verwendung einer Sterilisationskammer mit besonders hoher Nachweisgenauigkeit ausgelegt. Dazu wird der Prüfkörper 1 gemeinsam mit den zu sterilisierenden Instrumenten oder Materialien in die Sterilisationskammer eingebracht. Im ersten Bearbeitungsschritt, in dem die Sterilisationskammer evakuiert wird, werden auch der Gassammelraum 8 sowie der Detektorraum 4 evakuiert. Anschließend, wenn die Sterilisationskammer mit Dampf als Sterilisiermittel befüllt wird, strömt der Dampf über die Endmündung 10 auch in den Gassammelraum 8 und über diesen in den Detektorraum 4 ein. Eventuell noch vorhandene Restluft, die durch die Bildung von Luftpolstern eine zuverlässige Sterilisation der Instrumente oder Materialien verhindern könnte, wird dabei über den Gassammelraum 8 in den Detektorraum 4 hineingedrückt. Somit erfolgt eine Aufkonzentration der Restluft im Bereich des Detektors 2, so dass eine vollständige Benetzung des im Detektor 2 angeordneten Indikators 6 mit dem als Sterilisiermittel vorgesehen Dampf unterbleibt.

In sukzessiv erfolgenden weiteren Verfahrensschritten, bei denen in der Art eines fraktionierten Vakuums erneut ein Unterdruck in der Sterilisationskammer erzeugt und diese anschließend mit Dampf als Sterilisiermittel befüllt wird, erfolgt ein sukzessiv zunehmender Austrag der Restluft aus der Sterilisationskammer. Dadurch erfolgt eine zunehmend zuverlässige und durchgängige Benetzung sämtlicher inneren Oberflächen der Instrumente, die analog auch zu einer zunehmend besseren Benetzung der Innenoberfläche des Gassammelraums 8 führt. Falls eine ausreichende Penetration des Sterilisiermittels in den Prüfkörper 1 hinein erfolgt, kann eine durchgängige Benetzung auch des Indikators 6 festgestellt werden. Wenn dies eintritt, wird die Sterilisationsmaßnahme als erfolgreich angesehen.

Der Indikator 6, der im Ausführungsbeispiel nach Fig. 1 ein Volumen von etwa 120 µl einnimmt und den Detektorraum 4 somit annähernd zur Hälfte ausfüllt, könnte als Sensor zur Ermittlung eines physikalischen Messwerts, beispielsweise der Temperatur oder des Drucks, ausgelegt sein. Im Ausführungsbeispiel nach Fig. 1 ist als Indikator 6 jedoch ein Bio- oder Chemo-Indikator vorgesehen. Bei der Ausgestaltung als Bio-Indikator ist der Indikator 6 dabei an seiner Oberfläche mit keimfähigen Kulturen versetzt, die bei einer ordnungsgemäßen Benetzung mit Dampf als Sterilisiermittel abgetötet werden. Bei dieser Ausgestaltung wird der Indikator 6 also derart eingesetzt, dass nach erfolgter Sterilisation eine Überprüfung auf noch vermehrungsfähige Keime vorgenommen wird. Falls solche festgestellt werden, wird auf unzureichende Sterilisation geschlossen.

Bei einer Ausgestaltung des Indikators 6 als Chemo-Indikator ist dieser derart ausgebildet, dass er bei einer Benetzung seiner Oberfläche mit Dampf als Sterilisiermittel unter Einwirkung von Temperatur über eine vorgegebene Zeit seine Farbe ändert, so dass durch optische Kontrolle direkt auf eine durchgängige Benetzung geschlossen werden kann. Bei Nicht-Benetzung erfolgt kein oder ein anderer Farbumschlag.

Der in Hohlkörperbauweise ausgeführte Prüfkörper 1 ist zur Sicherstellung besonders zuverlässiger Prüfungsergebnisse mit hoher Reproduzierbarkeit und bei besonders gering gehaltenem Aufwand ausgelegt. Dazu ist der Prüfkörper 1 als Einwegprodukt ausgelegt. Diese Auslegung manifestiert sich unter anderem darin, dass hinsichtlich der Auswahl der eingesetzten Materialien, der Dimensionierung der einzelnen Komponenten und dergleichen der Prüfkörper 1 für einen lediglich einmaligen Gebrauch und nicht für eine Wiederverwendung vorgesehen ist.

Der Detektorraum 4 und/oder der Gassammelraum 8 können jeweils aus Metall gebildet sein. Im Ausführungsbeispiel nach Fig. 1 sind der Detektorraum 4 und der Gassammelraum 8 jedoch jeweils als Schlauchstück ausgebildet. Je nach einzustellenden Prüfbedingungen und dergleichen können die den Detektorraum 4 und den Gassammelraum 8 bildenden Schlauchstücke dabei insbesondere hinsichtlich der Materialwahl, der Dimensionierung und/oder des Strömungsquerschnitts für das Gas unterschiedlich voneinander ausgeführt sein. Im Ausführungsbeispiel nach Fig. 1 ist der Prüfkörper 1 jedoch für eine besonders einfach gehaltene Bauweise ausgelegt, wobei der Detektorraum 4 und der Gassammelraum 8 in der Art einer einstückigen Ausführung durch einen gemeinsamen Schlauchkörper 14 gebildet sind. Der Schlauchkörper 14 ist dabei im Ausführungsbeispiel aus transparentem Kunststoff ausgeführt, so dass durch optische Auswertung, insbesondere durch Betrachtung, des Indikators 6 eine Ermittlung der Prüfungsergebnisse ermöglicht ist.

Eine besonders kostengünstige Herstellung des Prüfkörpers 1 nach Fig. 1 ist möglich, indem der Indikator 6 in ein Schlauchende des Schlauchkörpers 14 eingebracht und dort eingebettet wird, wobei anschließend das entsprechende Schlauchende 16 verschweißt wird. Dabei entsteht die in Fig. 1 angedeutete Schweißnaht 18.

Aufgrund der Ausführung des Prüfkörpers 1 als Einwegprodukt ist eine zerstörungsfreie Entnahme des Indikators 6 aus dem Detektorraum 4 nach erfolgter Verifikationsmaßnahme weder vorgesehen noch erforderlich. Stattdessen wird bei der Entnahme des Indikators 6 das den Detektorraum 4 bildende Gehäuse aufgebrochen, wobei anschließend der verbleibende Rest des Prüfkörpers 1 einer Entsorgung zugeführt werden kann. Um die Entnahme des Indikators 6 zu erleichtern, weist das den Detektorraum 4 bildende Gehäuse eine Sollbruchstelle 20, also insbesondere eine geeignet vorgegebene Schwachstelle im Wandmaterial, auf.

Zur Erhöhung der Nachweisgenauigkeit kann der Gassammelraum 8 und ggf. auch der Detektorraum 4 von einer Wärme isolierenden Ummantelung umgeben sein.

In Fig. 2 ist eine alternative Ausführungsform des Prüfkörpers 1' gezeigt, bei der als Indikator 6 auf eine transparente Innenwand des den Detektorraum 4 bildenden Schlauchstücks aufgebrachte Indikatorfarbe vorgesehen ist. Bei der Herstellung eines derartig ausgestalteten Prüfkörpers 1' wird vorzugsweise auf das zur Bereitstellung des Detektorraums 4 vorgesehene Schlauchstück des Schlauchkörpers 14 eine Suspension eines Chemo-Indikators aufgebracht und anschließend getrocknet. Bei einer derartigen, mit besonders einfachen Mitteln und gering gehaltenem Aufwand herstellbaren Ausführung des Prüfkörpers 1' bilden der Indikator 6 und das den Detektorraum 4 bildende Schlauchstück des Schlauchkörpers 14 somit eine mechanisch integrierte Einheit, bei der der Indikator 6 nicht ohne weiteres vom entsprechenden Schlauchstück ablösbar ist. Zur Begutachtung, Archivierung und Protokollierung von Verifizierungsmaßnahmen kann in diesem Fall eine Aufbewahrung des Indikators 6 gemeinsam mit dem ihn tragenden Schlauchstück vorgesehen sein.

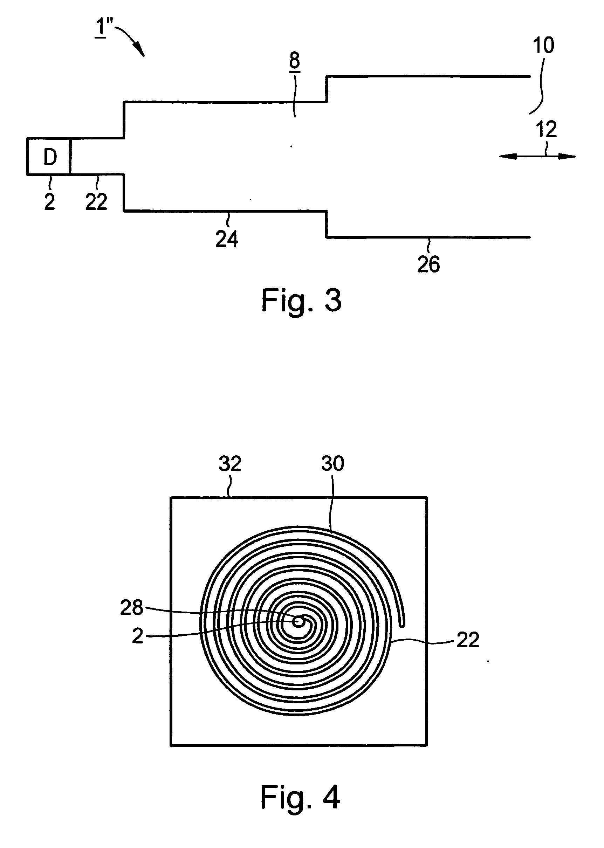

Im Ausführungsbeispiel nach Fig. 3 ist der Prüfkörper 1" für eine besonders kompakte Bauweise bei gleichzeitig besonders hoher Nachweisempfindlichkeit hinsichtlich der Penetrationseigenschaften der zu simulierenden Instrumente oder Gerätschaften ausgebildet. Dazu ist in diesem Ausführungsbeispiel der Gassammelraum 8 mehrstufig ausgeführt, wobei eine dem Detektor 2 unmittelbar benachbarte erste Stufe 22 gasseitig mit weiteren Stufen 24, 26 in Reihe geschaltet ist. Selbstverständlich können in Ergänzung einer derartigen dreistufigen Ausführung auch noch weitere Stufen hintereinandergeschaltet sein, oder es kann eine zweistufige Ausführung vorgesehen sein.

Durch die mehrstufige Ausgestaltung des Gassammelraums 8 mittels der gasseitigen Vorschaltung der zweiten und dritten Stufe 24, 26 vor die erste Stufe 22, und durch eine geeignet gewählte Dimensionierung der Stufen 22, 24, 26 ist beim Prüfkörper 1" sichergestellt, dass die für den Prozess schädliche Restluft im unmittelbaren Bereich der ersten Stufe 22 gezielt aufkonzentriert wird. Diese Aufkonzentration erfolgt insbesondere durch die gasseitig vorgelagerten Stufen 24, 26, die als Kondensationszone für das Sterilisiermittel dienen und eine selektive lokale Auskondensation des Sterilisiermittels unmittelbar vor dem Eintrittsbereich in die erste Stufe 22 bewirken. Durch die damit erreichte Aufkonzentration der Restluft oder anderer nicht kondensierbarer Gase erfolgt ein nachhaltiger und besonders zuverlässiger Nachweis der Restluft oder andere nicht kondensierbare Gase, so dass der Prüfkörper 1" mit einer besonders hohen Nachweisgenauigkeit betrieben werden kann. Um dies zu gewährleisten, nehmen Strömungsquerschnitt und Volumen der Stufen 24, 26 zwischen den benachbarten Stufen 24, 26 in Strömungsrichtung zum Detektor 2 hin signifikant ab.

Ein nach dem Aufbauschema gemäß Fig. 3 aufgebauter Prüfkörper 1'" ist in Fig. 4 geschachtelt gezeigt. Dessen Detektor 2 umfasst ein zur Bildung des Detektorraums 4 und zur Aufnahme des Indikators 6 vorgesehenes, durch Spritzgusstechnik hergestelltes Detektorgehäuse 28, das mit einem zur Bildung der ersten Stufe 22 des Gassammelraums 8 vorgesehenen Schlauchstück 30 verbunden ist. Beim Prüfkörper 1'" nach Fig. 4 ist die erste Stufe 22 des Gassammelraums 8 in der Art einer geschachtelten Bauweise innerhalb der zweiten Stufe 24 des Gassammelraums 8 geführt. Der Prüfkörper 1'" ist in diesem Fall jedoch für eine besonders hohe Nachweisempfindlichkeit bei besonders kompakter Bauweise ausgeführt. Um die für eine hohe Nachweisempfindlichkeit günstige vergleichsweise große Kanallänge der ersten Stufe 22 auch bei kompakter Bauweise herzustellen, ist die erste Stufe 22 in dieser Ausführungsform in der Art einer Wendel oder gewickelten Spirale innerhalb des die zweite Stufe 24 umschließenden Außengehäuses 32 geführt.

### Bezugszeichenliste

- 1, 1', 1 ", 1"': Prüfkörper
- 2: Detektor
- 4: Detektorraum
- 6: Indikator
- 8: Gassammelraum
- 10: Endmündung
- 12: Doppelpfeil
- 14: Schlauchkörper
- 16: Schlauchende
- 18: Schweißnaht
- 20: Sollbruchstelle
- 21: Deckel
- 22,24,26: Stufen
- 28: Detektorgehäuse
- 30: Schlauchstück
- 32: Außengehäuse

## Patentansprüche

1. Prüfkörper (1, 1', 1'', 1''') in Hohlkörperbauweise, insbesondere zur Überprüfung der Penetrationseigenschaften eines Sterilisiermittels in einem Sterilisationsprozess, der einen mit einem Indikator (6) versehenen Detektorraum (4) und einen mit diesem gasseitig verbundenen, von einem Kunststoff-Schlauch gebildeten Gassammelraum (8) aufweist, und der derart als Einwegprodukt ausgelegt ist, dass er bei einer Entnahme des Indikators (6) irreversibel zerstört ist wobei der Indikator (6) an einem der Schlauchenden eingebettet und das Schlauchende zur Bildung des Detektorraums (4) verschweißt ist.

2. Prüfkörper (1, 1', 1'', 1''') nach Anspruch 1, dessen Gassammelraum (8) als Spirale aufgewickelt ist.

3. Prüfkörper (1, 1', 1'', 1''') nach Anspruch 1 oder 2, bei dem das den Detektorraum (4) bildende Gehäuse eine Sollbruchstelle (20) aufweist.

4. Prüfkörper (1, 1', 1'', 1''') nach einem der Ansprüche 1 bis 3, dessen Gassammelraum (8) mehrstufig ausgeführt ist, wobei der Querschnitt und/oder das Volumen jeder Stufe (22, 24, 26) zwischen benachbarten Stufen (22, 24, 26) in Richtung zum Detektorraum (4) abnehmen.

5. Prüfkörper (1, 1', 1'', 1''') nach einem der Ansprüche 1 bis 4, dessen Gassammelraum (8) mit einer wärmeisolierenden Ummantelung versehen ist.

6. Prüfkörper (1, 1', 1'', 1''') nach einem der Ansprüche 1 bis 5, bei dem eine Indikatorfarbe auf der Innenseite einer transparenten Wand des den Detektorraum bildenden Gehäuses als Detektor vorgesehen ist.

7. Prüfkörper (1, 1', 1 ", 1''') nach einem der Ansprüche 1 bis 6, bei dem als Indikator (6) ein Chemo-, ein Bio- und/oder ein physikalischer Indikator vorgesehen ist.

8. Prüfkörper (1, 1', 1'', 1''') nach einem der Ansprüche 1 bis 7, bei dem der Schlauch aus transparentem Kunststoff gebildet ist.

9. Verfahren zur Herstellung eines Prüfkörpers (1, 1', 1", 1''') nach einem der Ansprüche 1 bis 8, bei dem auf die Innenwand eines zur Bereitstellung eines Detektorraumes (4) vorgesehenen Schlauchstücks eine Suspension eines Chemo- oder Bio-Indikators aufgebracht und anschließend getrocknet wird.

## Claims

1. Test device (1, 1', 1'', 1''') in hollow construction, especially to test the penetration characteristics of a sterilization agent in a sterilization process, with a detector volume (4) provided with an indicator (6) and a gas-collection-volume (8) consisting of a plastic tube, which is connected to the detector volume (4) at the gas side, that is designed as disposable product so that it is irreversibly destroyed after extraction of the indicator (6), whereas the indicator (6) is embedded at one of the tube ends ad the tube end is welded to build the detector volume (4).

2. Test device (1, 1', 1 ", 1''') according to claim 1, whose gas-collection-volume is winded as a helix.

3. Test device (1, 1', 1'', 1''') according to claim 1 or 2, whose case building the detector volume (4) has a predetermined breaking point (2).

4. Test device (1, 1', 1", 1''') according to one of the claims 1 to 3, whose gas-collection-volume (8) is multi-stage designed, whereas the cross-section and/or the volume of each stage (22, 24, 26) decreases between the adjacent stages (22, 24, 26) in direction of the detector volume (4).

5. Test device (1, 1', 1", 1''') according to one of the claims 1 to 4, whose gas-collection-volume (8) is provided with a heat insulating cover.

6. Test device (1, 1', 1", 1''') according to one of the claims 1 to 5 with an indicator colour as detector at the inside of a transparent wall of the case building the detector volume.

7. Test device (1, 1', 1'', 1''') according to one of the claims 1 to 5 with a chemical, biological and/or physical indicator as detector.

8. Test device (1, 1', 1", 1'") according to one of the claims 1 to 7, with a tube of transparent plastic material.

9. Procedure to produce a test device (1, 1', 1", 1''') according to one of the claims 1 to 8, where at the inner wall of a piece of tube, which is provided to function as a detector volume (4), a suspension of a chemical or biological indicator is applied and dried afterwards.

## Revendications

1. Corps de contrôle (1, 1', 1 ", 1"') sous forme de corps creux, destiné en particulier au contrôle des caractéristiques de pénétration d'un produit stérilisant dans un processus de stérilisation, qui comprend un volume détecteur (4) pourvu d'un indicateur (6) et un collecteur de gaz (8) formé par un tuyau en matière plastique relié avec lui du côté du collecteur de gaz, corps de contrôle conçu comme produit jetable en sorte qu'il est détruit de manière irréversible si l'indicateur (6) est retiré, cet indicateur (6) étant intégré à une extrémité du tuyau et le bout du tuyau étant soudé pour former le volume détecteur (4).

2. Corps de contrôle (1, 1', 1'', 1''') selon spécification 1, dont le collecteur de gaz (8) est enroulé en forme de spirale.

3. Corps de contrôle (1, 1', 1", 1"') selon spécification 1 ou 2, dans lequel le boîtier formant le volume détecteur (4) comprend un point de rupture prédéterminé (2).

4. Corps de contrôle (1, 1', 1", 1"') selon l'une des spécifications 1 à 3, dont le collecteur de gaz (8) comprend plusieurs étapes telles que section et/ou volume de chaque étape (22, 24, 26) entre les étapes voisines (22, 24, 26) va/vont en décroissant en direction du volume détecteur (4).

5. Corps de contrôle (1, 1', 1", 1''') selon l'une des spécifications 1 à 4, dont le collecteur de gaz (8) est enrobé d'une gaine isolante.

6. Corps de contrôle (1, 1', 1", 1 "') selon l'une des spécifications 1 à 5, dans lequel un colorant indicateur est prévu comme détecteur sur la surface intérieure d'une paroi transparente du boîtier constituant le volume détecteur (4).

7. Corps de contrôle (1, 1', 1'', 1''') selon l'une des spécifications 1 à 5, pour lequel est prévu comme detecteur (6) un indicateur chimique, un indicateur biologique et/ou un indicateur physique.

8. Corps de contrôle (1, 1', 1'', 1''') selon l'une des spécifications 1 à 7, dans lequel le tuyau est fabriqué en matière plastique transparente.

9. Procédé pour la fabrication d'un corps de contrôle (1, 1', 1'', 1''') selon l'une des spécifications 1 à 8, dans lequel un indicateur chimique ou biologique en suspension est placé sur la paroi intérieure d'un morceau de tuyau prévu pour la mise à disposition d'un volume détecteur (4), suspension destinée à être séchée ensuite.
